Europäisches Patentamt

(19) European Patent Office

Office européen des brevets

(11) Publication number: **0 448 609 B1**

(12) **EUROPEAN PATENT SPECIFICATION**

(45) Date of publication of patent specification :
18.11.93 Bulletin 93/46

(51) Int. CI.⁵ : **C12N 15/10, C12Q 1/68**

(21) Application number : **90900787.4**

(22) Date of filing : **21.11.89**

(86) International application number :
**PCT/EP89/01417**

(87) International publication number :
**WO 90/06043 14.06.90 Gazette 90/14**

(54) **IN VITRO MUTAGENESIS.**

(30) Priority : **21.11.88 GB 8827167**

(43) Date of publication of application :
**02.10.91 Bulletin 91/40**

(45) Publication of the grant of the patent :
**18.11.93 Bulletin 93/46**

(84) Designated Contracting States :
**AT BE CH DE ES FR GB IT LI LU NL SE**

(56) References cited :
**EP-A- 0 223 618**
**EP-A- 0 258 017**
**EP-A- 0 265 244**
**EP-A- 0 265 293**
**Nucleic Acids Research, vol. 16, no. 7, 1988, IRL Press Ltd, (Oxford, GB), S. Stahl et al.: "Solid phase DNA sequencing using the biotin-avidin system", pp. 3025-3038**

(56) References cited :
**DNA, vol. 3, no. 6, 1984, Mary Ann Liebert, Inc., Publishers, M.J. Zoller et al.: "Laboratory Methods. Oligonucleotide-directed mutgensis: a simple method using two oligonucleotide primers and a single-stranded DNA template", pp. 479-487**

(73) Proprietor : **DYNAL A.S.**
**P.O. Box 158 Skoyen**
**N-0212 Oslo 2 (NO)**

(72) Inventor : **HORNES, Erik**
**Lilleakeron 9B**
**N-0283 Oslo 2 (NO)**
Inventor : **KORSNES, Lars**
**Monolittveien 12**
**N-0375 Oslo 3 (NO)**

(74) Representative : **Holmes, Michael John et al**
**Frank B. Dehn & Co. Imperial House 15-19 Kingsway**
**London WC2B 6UZ (GB)**

EP 0 448 609 B1

EP 0 448 609 B1

## Description

This invention relates to in vitro mutagenesis and provides a simple and rapid method and kit for effecting site specific mutagenesis.

Conventionally site specific mutagenesis has been effected by isolating, and where necessary denaturing, the parent gene or other DNA sequence to produce single stranded DNA. Alternatively, single or double stranded DNA may be incorporated into phage M13 and cloned to produce single stranded M13. An oligonucleotide primer having the required base alteration or alterations and which is long enough to bind to the selected site in spite of the mis-matched base pair or pairs is added to the single stranded DNA. Appropriate ligases and polymerases, together with a supply of nucleotides are added. A new DNA strand is formed having the required base alteration(s) (see Zoller, M.J. & Smith, M. (1983) Meth. Enzymol 100, 468-500).

There is a need to simplify the procedure for site-specific mutagenesis and to increase the speed at which such mutations can be made.

Furthermore, the conventional technique requires a specially constructed bacterial host strain to minimise growth of the parent plasmid since it is difficult to remove the parent DNA from the altered DNA prior to cloning.

The invention provides a method for <u>in vitro</u> mutagenesis of a nucleic acid to produce single stranded DNA containing one or more base alterations, which includes the steps of:

a) preparing an insoluble support carrying a nucleic acid to be mutagenised;

b) adding a DNA oligonucleotide primer, which has the desired base alteration(s) but sufficient homology to hybridise with the appropriate site on the said first nucleic acid and allowing the primer to bind thereto;

c) where necessary by hybridising a further primer corresponding to the end of the nucleic acid at which synthesis is to start and adding a polymerase or a reverse transcriptase, and nucleotides to form a DNA strand having the desired alteration(s) and including a ligase to ligate the said DNA strand to the primer; and

d) separating the first nucleic acid from the synthesised DNA strand.

The nucleic acid to be mutagenised may be attached to the insoluble support by hybridisation to a nucleic acid probe attached to the support, which probe can serve as a primer for DNA synthesis, or the nucleic acid may be directly attached to the support.

Where the template nucleic acid is attached by hybridisation, the synthesised strand normally becomes covalently bonded to the probe and the synthesised DNA cannot readily be removed from the particles. It is useful therefore, where double stranded DNA is to be synthesised, to provide an RE site preferably near the region of the nucleic acid intended for subsequent cloning, to enable the double stranded DNA to be removed.

Where the nucleic acid is directly attached, without there being a primer sequence at the point of attachment, a primer will be added to initiate synthesis and in this case the synthesised DNA strand can be removed from the template by denaturisation, separated from the template bound to the support, e.g. by removal of the supernatant, and then if desired the second DNA strand can be synthesised in solution.

The synthesis of any second DNA strand to form double stranded DNA will require addition of a suitable primer corresponding to the 3'-terminal region of the initially synthesised DNA strand.

Often the nucleic acid to be mutagenised is only available in very small quantities and it may often advantageously be amplified by the PCR (Polymerase Chain Reaction) technique. In the case of DNA, the unamplified dsDNA is denatured and primers are annealed to both the coding and the non-coding strand. The primers are preferably those corresponding to the 5'-terminal sequences of the DNA so that on extension of the primer with a polymerase, the whole DNA sequence of each strand will be replicated. The double stranded DNA so produced is then denatured by raising the temperature followed by rapid cooling. An excess of the primer molecules is present and are annealed to the newly formed coding and non-coding strands. Extension using polymerase produces further double stranded DNA. The temperature cycling can be repeated many times, thereby producing a large number of copies of the DNA. Preferably, the polymerase used is one which can withstand the highest temperature of the cycle, commonly the <u>Tag</u> polymerase; otherwise there is a need to separate the polymerase from the nucleic acids before each heating step or replenish the polymerase after each cooling step. Such PCR amplification provides target DNA incorporating the primers which are used. One of these may be attached to the insoluble support, e.g. magnetic particles, or may be provided with means for subsequent attachment e.g. biotin groups, so that the target DNA can readily be obtained attached to the support for mutagenesis according to the invention.

Double stranded DNA is normally synthesised in order to permit subsequent cloning by incorporation into a plasmid or other vector. However, where the template nucleic acid is directly attached to the support, it is possible to avoid synthesis of double stranded DNA prior to cloning the mutagenised DNA. The template nucleic acid may be provided with terminal regions homologous with those of one strand of a linearised vector so that the synthesised ss DNA has terminal regions which hybridise with the respective ends of the linearised vector strand. After synthesis of the mutagenised ss DNA strand, this may be liberated, e.g. by melting, and contacted with the linearised vector strand to produce a cyclic product comprising two single stranded regions derived from the mutagenised cDNA and the vector and two double stranded regions where the terminal regions overlap and hybridise. After annealing, such a cyclic product may surprisingly be used directly to transform a suitable host such as E. coli. The native polymerases in the host organism, surprisingly, extend each of the incomplete strands of the cyclic product to produce a double stranded plasmid capable of replication.

In one version of this strategy, a plasmid in double stranded form containing the DNA sequence to be mutagenised has two RE sites flanking this DNA sequence and two further RE sites inside these, each separated from the outer RE sites. The plasmid is cut at one of the inner RE sites and biotinylated followed by restriction at the other inner RE site. This provides a linearised double stranded vector which is then attached to an insoluble support coated with avidin or streptavidin. One strand of the linearised vector is thus attached to the support while the other is not and can then be brought into solution by denaturation. The shorter sequence comprising the excised DNA sequence to be mutagenised is rejected. A further sample of the original plasmid can then be cut at one of the outer RE sites flanking the initially cut RE sites and the sticky end biotinylated; final cutting of the fourth RE site provides the template DNA (for mutagenesis) in double stranded form, one end of one strand of which is biotinylated and may be attached to a support. Removal of the unattached strand by denaturation leaves the biotinylated strand immobilised. The RE sites must be chosen to permit the immobilised strand to correspond to the liberated vector strand, so that the mutagenised cDNA strand to be synthesised is complementary to the vector strand in the flanking sections between the RE sites. A primer corresponding to the biotinylated flanking region is then added, together with a mutagenesis primer incorporating the desired DNA modification. Extension, using a polymerase, and ligation provides a single stranded mutagenised cDNA strand which can be split off from the template by denaturation.

It will be appreciated that the terminal regions of the single stranded linearised vector will be complementary to the terminal flanking regions of the mutagenised DNA sequence and when brought into contact and annealed, a cyclic product will be formed which, as indicated above, can be transformed into a host and converted into a complete, replicating plasmid.

According to a still further strategy, a standard linearised single stranded vector can be prepared in solution as described above. A further plasmid containing a DNA sequence to be mutagenised is flanked by sequences corresponding to the terminal regions of the single stranded linearised vector. The further plasmid can be subjected to at least one or two cycles of PCR amplification using primers flanking the target DNA sequence (to be mutagenised), these primers being homologous with the terminal sequences of the linearised vector. One of the primers is provided with means for attachment to a support (e.g. a biotin group) or is already attached to the support. Chain extension provides, after a final strand separation, the target DNA in single stranded form linked at one end to a support. Hybridisation to a further primer at the 3'-end to initiate chain extension and a mutagenesis primer incorporating the desired mutation, permits synthesis, in the presence of a polymerase, of a cDNA strand incorporating the mutation and flanked by terminal sequences complimentary to those of the linearised vector. Strand separation, e.g. by treatment with alkali, liberates the mutagenised strand into solution while the template is immobilised and thus readily separated. The mutagenised ss cDNA may then be contacted with the linearised vector and annealed to give a cyclic product as above.

It will be appreciated that in any of the above systems, the biotin/avidin or streptavidin affinity coupling may be replaced by other such coupling using a relatively small molecule and a binding partner, e.g. an antibody therefor.

Direct attachment of the DNA to the support may, for example, be by affinity, e.g. via a biotin-avidin bond or covalently as indicated below.

An advantage of the present invention is that the template is readily removed completely from the synthesised DNA, thus avoiding contamination with unmutated DNA.

As indicated above, the probes can be DNA moieties which will hybridise with RNA or DNA. These include oligo-dT, which will hybridise with the poly A 'tails' universally present on native mRNA, and probes comprising specific DNA sequences which hybridise with specific sequences in target DNA and RNA molecules. Each probe may consist of a directly attached single stranded DNA which may be oligo-dT or a specific DNA sequence or it may be attached to the insoluble support via a double stranded piece of DNA.

A particularly useful form of probe for use where one wishes to isolate mRNA for mutation and subsequent cDNA synthesis is a DNA sequence in which the 3' end overlaps and is hybridised to a region near the 5' end, leaving the remainder of the 5'-terminal region as a sticky end to hybridise with the target nucleic acid. If a functional group such as an amino group is present in a position distal from the sticky end, the loop may be covalently attached to the insoluble support e.g. via carboxyl groups. Alternatively, a biotin group may be attached to the loop and thus bind the probe to a streptavidin coated support. DNA having a terminal region corresponding to the sticky end will thus have the possibility of being ligated to the adjacent part of the loop if it is required to secure the DNA covalently. RE sites can be provided in the overlap region of the probe for subsequent detachment of the DNA.

Where method of the invention is used to mutagenise mRNA material, e.g. from a cell lysate, the probe is advantageously oligo-dT, that is a relatively short chain of deoxythymidine units, e.g. from 20 to 200 bases. Such a chain may be readily and cheaply prepared by enzymic polymerisation of deoxythymidine units or using a DNA synthesiser, e.g. from 20 to 200 bases, conveniently about 25 bases.

Where the target nucleic acid is a specific nucleic acid or a family of nucleic acids having a known conserved region, the probe/primer will comprise a specific oligonucleotide sequence which may be synthesised by conventional methods. Where the inert support carries hydroxyl groups, it is possible to synthesise directly onto the support.

To avoid random hybridisation of unwanted nucleic acid and to complete the removal of the remaining components of the hybridisation solution, the insoluble support is preferably washed at least once after separation. To remove nucleic acid bound by random partial homology, the washing may be carried out under stringent conditions, either by increasing the temperature or by using a lower salt concentration than that used in hybridisation, e.g. 0.5M sodium chloride or an equivalent solution.

Stringency is normally calculated according to the probe length and G:C content. If the homology between the probe oligonucleotide and the target nucleic acid isinexact, washing should be carried out under less stringent conditions. In general, washing should be carried out at a temperature 12°C below the melting temperature of the duplex ($T_m$). The approximate $T_m$ may be conveniently calculated according to the following relationships (taken from Maniatis, T. et al (1982) Molecular Cloning; a laboratory manual pages 388-389).

(a) $T_m$ = 69.3 + 0.41 ·(G+C)% - 650/L L equals the average length of the probe in nucleotides.

(b) The $T_m$ duplex DNA decreases by 1°C with every increase of 1% in the number of mismatched base pairs.

(c) $(T_m)u_2 - (T_m)u_1 = 18.5 \log_{10}\frac{u_2}{u_1}$

where $u_1$ and $u_2$ are the ionic strengths of two solutions.

For small oligonucleotides, the melt temperature may be approximated in degrees centigrade as follows:

$$T_m = 2 \times (\text{number of A + T residues}) + 4 \times (\text{number of G + C residues})$$

The hybridisation reaction is preferably effected in a 1M sodium chloride solution or an equivalent solution known in the art. (See Nucleic Acid Hybridisation, B D Hames and S J Higgins, IRL Press, 1985).

The target nucleic acid may require an initial treatment step before performance of the method according to the invention. For example, if the target nucleic acid is dsDNA an initial melting step followed by rapid cooling will be necessary so as to provide ssDNA. (Determination of the melt temperature Tm is discussed above). If it is desired to mutagenise a particular mRNA from a pool of mRNA then, due to the lower stability of RNA compared with DNA it may be preferable to first synthesize cDNA for all mRNA species in the pool before selecting the target nucleic acid with the specific probe. If the target nucleic acid is of relatively low abundance then it is preferable to amplify it using PCR if, of course, two spaced specific sequences are known.

Probe and primer oligonucleotides may be prepared by using any of the commercially available DNA synthesis devices, e.g. those available from Applied Biosystems, Inc. (850-T Lincoln Center Drive, Foster City, CA 94404).

In conventional methods enzymic operations are often carried out in the same unchanged buffer which is thus not optimised for each reaction. However, the method according to the invention allows one to change buffers and the like and thereby optimise the production of cDNA containing the desired mutation. Further, in conventional ss cDNA synthesis, the ratio of nucleotide reagents to mRNA is usually kept approximately stoichiometric in order to avoid contaminating succeeding stages with excess reagent. The ease and speed of washing, which comes from immobilisation of target nucleic acid according to the invention, permits excess reagents to be used, with a consequent increase of efficiency.

The insoluble support may take a variety of forms, for example microtitre wells, filters made from materials such as cellulose or nylon, or particles including, for example, sephadex or sepharose beads or polystyrene latex particles. It is a preferred feature of the invention to use magnetic particles which may be aggregated onto a surface and then be readily re-dispersed for a subsequent treatment step, e.g. by physical agitation.

Advantageously the particles are monodisperse and/or superparamagnetic. Both these properties greatly assist the kinetics of reactions in which the particles are involved. It is a surprising feature of the invention that the probes carried by the particles react in the reactions virtually as rapidly as if free in solution. For example, hybridisation and purification can be effected in less than 15 minutes which contrasts sharply with the 2 hours required for hybridisation and purification of mRNA onto poly-dT affinity column. By using monodisperse particles, that is particles of substantially the same size, the reaction rate and other parameters are particularly uniform. By using superparamagnetic particles (that is particles containing sub-particles of ferromagnetic material which are smaller than the domain size required to maintain permanent magnetism), one can avoid magnetic aggregation or clumping of the particles during reaction, thus again ensuring uniform and rapid reaction kinetics.

The preferred magnetic particles for use in this invention are monodisperse superparamagnetic beads produced according to EP 83901406.5 (Sintef), the disclosure of which is incorporated herein by reference. In these beads, the iron is very uniformly distributed and provides a very uniform response to a magnetic field which is important in designing a reproducible procedure, particularly for automation, since all the beads move at the same speed. Furthermore, since a reproducible amount of iron can be incorporated in each particle, this can be adjusted to a relatively low level which permits the specific gravity of the particles to be in the range specified below. In the case of prior, less regular products, small particles either had too little iron to counteract Brownian forces when a magnet was applied or the specific gravity of the material led to undesirable sedimentation of the larger particles. Some automated systems use magnetic fields to restrain the particles within a reaction zone while solutions are passed through; uniform magnetic and rheological properties are essential in magnetic particles for use in such a system.

The term "monodisperse" used herein is intended to encompass size dispersions having a diameter standard deviation of less than 5%.

We prefer to use beads having a specific gravity in the range 1.1 to 1.8 most particularly 1.2 to 1.5. In the monodisperse beads used in accordance with the invention, the specific gravity is, again, particularly uniform, leading to uniform and predictable kinetic characteristics.

Advantageously, the monodisperse particles are spherical beads of diameter at least 1 and preferably at least 2 microns, being preferably not more than 10 and more preferably not more than 6 microns in diameter e.g. about 3 microns. Smaller particles sediment more slowly and in some cases the sedimentation time may be long compared to the reaction time, thus avoiding the need for physical agitation. However, particles of mean diameter 0.1 to 1.5 microns including fine particles of much smaller diameter, as used in the prior art, behave unreliably in response to magnetisation.

The attachment of the probes to the particles may be by direct chemical bonding as well as affinity binding, by streptavidin/biotin complexes and the like.

For attachment of the probes, the magnetic particles may carry functional groups such as hydroxyl, carboxyl, aldehyde or amino groups. These may in general be provided by treating uncoated monodisperse, superparamagnetic beads, to provide a surface coating of a polymer carrying one of such functional groups, e.g. polyurethane together with a polyglycol to provide hydroxyl groups, or a cellulose derivative to provide hydroxyl groups, a polymer or copolymer of acrylic acid or methacrylic acid to provide carboxyl groups or an aminoalkylated polymer to provide amino groups. US Patent No. 4654267 describes the introduction of many such surface coatings.

Preferred coated particles for use in the present invention may be prepared by modification of the beads according to the US Patents 4336173, 4459378 and 4654267, the disclosure of which is incorporated herein by reference. Thus, for example, macroreticular porous polymer particles, prepared from styrene-divinylbenzene and with a diameter of 3.15um were treated with $HNO_3$ to introduce $-NO_2$ groups at the surface of the pores. Then the particles were dispersed in an aqueous solution of $Fe^{2+}$. The $Fe^{2+}$ is oxidised by the $-NO_2$ groups which leads to precipitation of insoluble iron oxy-hydroxy compounds inside the pores. After heating the iron exists as finely divided grains of magnetic iron oxides throughout the volume of the porous particles. The $NO_2$ groups are reduced by the reaction with $Fe^{++}$ to $NH_2$ groups.

To fill up the pores and to introduce the desired functional groups at the surfaces, different monomers are caused to polymerize in the pores and at the surface. In the case of a preferred type of particle, the surface carries -OH groups connected to the polymeric backbone through $-(CH_2CH_2O)_{8-10}$ linkages. Other preferred beads carry -COOH groups obtained through polymerization of methacrylic acid.

Thus, for example, the $NH_2$ groups initially present in the beads may be reacted with a diepoxide as described in US Patent No. 4654267 followed by reaction with methacrylic acid to provide a terminal vinyl grouping. Solution copolymerisation with methacrylic acid yields a polymeric coating carrying terminal carboxyl groups as in R452 beads referred to below. Similarly, amino groups can be introduced by reacting a diamine with the above product of the reaction with a diepoxide as in the R240, R442 and R469 beads, while reaction with a hydroxylamine such as aminoglycerol introduces hydroxy groups as in the M450 and L255 beads.

Dynabeads M450 (diameter 4.5 microns) which may be obtained from Dynal, Oslo, Norway have been coated with a monomeric epoxide, resulting in a mixture of epoxy and hydroxy groups. Contact with water however, converts the epoxy groups to hydroxy groups.

Dynabeads M-280 (diameter 2.8 microns) are polystyrene beads having hydroxyl groups which have been converted into tosyloxy groups by reaction with p-toluene sulphonyl chloride.

5

Using functionalised coatings of the above types, we have found the non-specific binding of DNA and/or RNA to be very low, particularly in the case of the carboxylated beads.

As indicated above, the probe and RE linker are preferably attached to the magnetic particles via carboxyl groups, the DNA being firstly provided with a 5'-terminal amino group which can be made to form an amide bond with the carboxyl using a carbodiimide coupling agent. 5'- attachment of DNA can also be effected using hydroxylated magnetic particles activated with CNBr to react with 5'- amino DNA.

The 3'-attachment of the oligonucleotide DNA can also be effected by chemical synthesis. Here again, the very uniform nature of the monodisperse particles provides uniform reaction rates particularly suited to synthesis in an automated synthesiser such as the Gene Assembler (Pharmacia AS). The magnetic particle needs to be provided initially with a hydroxyl or protected hydroxyl group. Dynabeads M-280 of Dynal A/S are well suited to this purpose. If necessary, however, other surface functions such as carboxyl could be used to attach a linker carrying a hydroxyl group or alternatively a 3'-attached nucleotide.

5'-Attachment may be effected by coupling of 5'-amino-oligonucleotides to tosyl-activated magnetic particles. The latter may be produced by tosylation of hydroxylated magnetic particles such as Dynabeads M-280 of Dynal A/S. Displacement of the tosyloxy group leaves the 5'-amino group directly attached to the magnetic beads.

Since biotin labelled nucleotides are commercially available, the 3'- end of DNA fragments can be labelled using DNA polymerase and these may be conveniently bound to avidin or streptavidin attached to the magnetic particles e.g. via a hydroxy group. The biotin label may be attached to the nucleotide by a spacer arm, such as one or more $\varepsilon$-aminocaproic acid moieties, to minimize steric hindrance.

In general, the functionalisation of the beads and subsequent attachment of probes is advantageously such that each magnetic particle carries $10^3$-$10^6$ probes. (1-300 pmols per mg). The uniform size of the magnetic particles is of advantage in ensuring uniform probe density when the probes are reacted with the particles. Uniform probe density is important in ensuring that all the probes behave in substantially the same way in the various procedures in which they are used.

It is a remarkable feature of the magnetic particles that enzyme activity appears to take place very close to the particle surface e.g. within 7 bases. Thus, if an RE site is present in a linker sequence as discussed hereinafter and if the probe is subsequently used as a primer, it is found that sscDNA and hence ds cDNA can be synthesised by the DNA polymerase past the RE site towards the bead surface and can thus itself readily be cleaved by the appropriate endonuclease. In the case of the carboxylated beads, it is found that the micro-surface of the beads is extremely irregular, presenting an unusually large surface area which may reduce steric hinderance to hybridisation and enzyme activity close to the surface. On the other hand the non-specific binding to such carboxylated beads is not increased.

According to a further feature of the invention we provide a kit for in vitro mutagenesis of nucleic acids comprising:

(a) an insoluble support adapted to capture a nucleic acid to be mutagenised;

(b) a polymerase or reverse transcriptase; and one or more of:

(c) a primer to initiate synthesis of cDNA;

(d) deoxynucleotides; and

(e) appropriate buffers.

In order to carry out PCR amplification prior to immobilisation of the target DNA for mutagenesis, the kit may also include, inter alia:

(i) two PCR primers one of which carries an affinity binding molecule capable of binding to said support; and

(ii) an alkaline solution for strand separation.

The following examples are given by way of illustration only:-

Example 1(a)

Carbodiimide (EDC) mediated attachment of 5' -NH$_2$ probes to carboxyl beads.

(a) The reaction used for attaching probes to carboxyl beads is as follows. Amino groups introduced at the 5'-end of the probes using a one-step reaction method described by Chu et al. (Chu, B.C.F., and Orgel, L.E. (1985) DNA 4, 327-331.), results in a greater nucleophilicity of the terminal primary amino group of the alkyl linker as compared to the amino functionalities of the bases. It was therefore expected that the carboxyl groups on the beads would react preferentially with these primary amino groups.

100 ug 5'-NH$_2$ modified probe in 600 ul of 0.1 M imidazole-buffer pH 7, 0.1 M EDC were added per mg of R452 carboxyl beads. The reaction mixtures were incubated for 20 hours at room temperature with gentle shaking.

(b) NH$_2$ modified probes were made using Applied Biosystem synthesizer and Aminolink II.

The coupling reactions were as follows:

10 μg 5'NH$_2$ modified probe in 100 μl of 0.1M imidazole buffer pH 7.0, 0.1M EDC was added per mg of R452 carboxyl beads. The reaction mixtures were incubated for 20 hours at room temperature on a roller mixer (Coulter) followed by washing in TE buffer containing 0.1M NaCl (4x).

Hydridization efficiency:

A range of beads with different amount of probe attached were tested in hybridization experiments with a complementary 25 mer polydT probe.

The beads covered the range 1-250 pmol probe attached per mg beads.

Increasing amounts of 25 mer polydA oligonucleotide hybridized with increasing amounts of probe attached. 193 pmol hybridized to beads with 250 pmol attached. However, when the target molecule was in the range of 1000 bp (control mRNA Promega Biosystems) there was no difference in hybridization efficiency between the bead with 100 pmol of attached probe compared with the more densely coupled beads.

## Example 2

Carbodiimide (EDC) mediated attachment of 5'-phosphate-probes to amino beads.

Probes were attached via a phosphoramidate linkage to 3 different amino beads by the method described by Ghosh et al. (Ghosh, S.S., and Musso, G.F. (1987) Nucl. Acids Res. 15, 5353 - 5372.). The amount of DNA attached to the different beads varied from 1.4-11.3 micrograms/mg.

The R469 beads which carry an amino group at the termini of an polyethylene glycol linker (8 atoms), bind a larger amount of probes than R240 beads which carry the amino group on a shorter linker (3 atoms). when the linker is made longer (number of atoms 20) as in the case of for the R442 beads, a decrease in the amount of probes bound to the beads is observed. This is probably due to secondary structures of the linkers which results in the terminal amino group becoming unavailable for coupling.

The amount of non-specifically bound DNA varies amoung the beads (7-30%) probably according to number of amino groups per unit of surface area. The R469 beads, which bind the largest amount of probes covalently (11 ug/mg), showed the lowest non-specific binding.

The acid lability of the phosphoramidate bond (Chu, B.C.F., Wahl, G.M., and Orgel, L.E. (1983) Nucl. Acids Res. 11, 6513 - 6529.) is used for measuring degree of end-attachment by acid hydrolysis. The amount end-attached probes varies between the different beads from 20-65%, and again, the R469 bead seems to be the preferable one with 65% of the probes end-attached.

We were able to attach twice as much probe material to the R469 beads by performing the reaction in imidazole buffer pH 7 for 3 hours at 50°C, instead of pH 6, for 24 hours at room temperature. An increase in molarity of EDC from 0.1 M to 0.2 M resulted in a 20% decrease in amount of probes on the R469 beads (data not shown).

## General Method

600 pmole (6 ug) of oligo A (36 mer) were dissolved in 1 ml of 0.1 M imidazole, pH 7, 0.1 M EDC and mixed with 5 mg of amino beads, and incubated for 3 hours at 50°C.

## Example 3

Coupling of 5'NH$_2$probes to tosyl activated beads

NH$_2$ groups were introduced at the 5' end of oligonucleotides using Applied Biosystems DNA Synthesizer 381A and Aminolink II to introduce the primary NH$_2$ group at the 5' end. Aminolink II is supplied from Applied Biosystems. After synthesis these amino modified oligonucleotides were used directly in the coupling experiment.

Tosyl activated M-280 beads are commercially available from DYNAL AS, Oslo.

Coupling procedure:

10 mg of tosyl activated beads were mixed with 50 μg NH$_2$ modified oligonucleotide in 100 μl 0.5M Na$_2$HPO$_4$ and incubated at 37°C for 20 hours on a roller mixer (Coulter) followed by washing in TE buffer containing 0.1M NaCl (4x).

## Example 4

### Direct synthesis

Dynabeads R 488 beads were used. They are the same beads as M-280 except that the diameter is 3.15 microns instead of 2.8 microns and they contain primary OH groups on the surface as in the M-280 beads.

Using the synthesizer (Pharmacia Gene Assembler) the 3' end of DNA will be attached to the surface.

Only small modifications were necessary to fit the 3.15 micron beads. In the standard small scale column from Applied Biosystems teflon filters with cut off at 3.0 microns were installed, the beads loaded and the column assembled.

Since this support does not contain dimethyltrityl (DMTr) groups and this machine stops if no such chemical is released in the first steps in the first cycle, small modifications in the start procedure were introduced. The synthesis was started using a standard ABI small scale column until the DMTr groups were released. Then the Gene Assembler was stopped manually and the modified column with magnetic beads was put into the Gene Assembler. The standard synthesis programme as recommended by the manufacturer was then followed. Deprotection was as recommended by Pharmacia. Direct synthesis was used to produce oligo(dT)$_{25}$ and the following sequence from the C region of the kappa light chain gene:

$$5'-TCACTGGATGGTGGGAAGATGGATACAGTTGGTGCA-3'.$$

## Example 5

### Materials and methods
### Magnetic beads

Dynabeads M-280 Streptavidin (Dynal A.S, Box 158, N-0212 Oslo) were used as solid phase. These are monodisperse superparamagnetic polymer particles with a diameter of 2.8 $\mu$m covalently coupled with Streptavidin. They have a surface area of 4.3 m$^2$/g.

### Biotin binding capacity

100 $\mu$l 6 x SSPE (Standard saline with phosphate and EDTA: Maniatis) containing 1 nmol $^{14}$C-Biotin (Amersham) was added to 0.5 mg beads (prewashed in 6 x SSPE) and placed on a roller mixer (Coulter) at room temperature for 15 minutes.

After two separate washes in 6 x SSPE the fraction of bound $^{14}$C-Biotin was measured by scintillation counting.

### Deoxyoligonucleotides

Deoxyoligonucleotides were synthesized on an Applied Biosystems 381A DNA synthesizer.

Chemicals were purchased from Applied Biosystems. 5'amino modified deoxyoligonucleotides were made using AminolinkII.

The immunoglobulin light kappa chain probe used was:

$$5'-TCACTGGATGGTGGGAAGATGGATACAGTTGGTGCA-3'.$$

### Biotinylation of probes

Biotin XNHS ester (Clontec N-succinimidyl of N-biotinyl $\varepsilon$-caproic acid) was used as recommended by the supplier.

0.1 $\mu$mol of NH$_2$-modified oligo(dT)$_{25}$ in 90 $\mu$l of water was added 10 $\mu$l labelling buffer (1M sodium bicarbonate/carbonate, pH 9.0) and vortexed.

Finally 25 $\mu$l Biotin XNHS ester (100 mg/ml) in dimethylformamide was added and incubated at room temperature overnight.

Excess labelling reagent and buffer was removed in a Sephadex G50 spin column.

The 5' Biotin oligo(dT)$_{25}$ was endlabelled using the fill in reaction by Klenow polymerase, $\alpha$-[$^{32}$P]-dTTP and oligo(dA)$_{25}$ as template. Excess label was removed using a Sephadex G50 spin column.

Preparation of oligo(dT) Dynabeads (T-beads)

200 µg Biotinylated oligo(dT)$_{25}$ (24 n mol) in 2.5 ml 6 x SSPE was mixed with 50 mg prewashed Dynabeads M-280 Streptavidin and incubated on a roller mixer for 15 minutes at room temperature.

After two washes in 6 x SSPE the beads were stored at 4°C in 6 x TE, 0.1 % SDS.

Oligonucleotide hybridization assay

In the standard assay to measure hybridization capacity of different batches of T-beads, 0.1 mg of the beads in an eppendorf tube was washed once with 6 x SSPE, 0.1 % SDS. A magnet rack (MPC-E, Dynal A.S., Oslo) was used to aggregate beads between each step.

After removal of the washing buffer, 50 µl hybridization solution (6 x SSPE, 0.1% SDS), containing 50 pmol of oligo(dA)$_{25}$ with trace amount (1-2 x $10^5$ cpm) $\alpha$-[$^{32}$P]-dATP-labelled oligo(dA)$_{25}$ was added.

After gentle mixing the tube was left to hybridize for two minutes at room temperature.

The hybridized beads were washed twice with 2 x SSPE, 0.1 % SDS at room temperature and the percentage of oligo(dA)$_{25}$ hybridized to the oligo(dT)$_{25}$ Dynabeads was measured in a scintillation counter.

Labelling of poly A mRNA tracer

1 µg 1200 bp mRNA with a 3'polyA$_{30}$ tail (Promega) was mixed with 2.5 pmol oligo(dT)$_{25}$ in 10 µl 5 x Klenow buffer, 1 u RNasin (Promega), 10mM DDT. After two minutes at room temperature 10 µCi$\alpha$-[$^{32}$P]-dATP, 1 u Klenow polymerase (Amersham) and water up to 50 µl were added and incubation continued for 60 minutes at 15°C. Excess $\alpha$-[$^{32}$P]-dATP was removed using a Sephadex spin column.

Buffers for poly(A) mRNA hybridization to Dynabeads M-280 Streptavidin coupled with oligo(dT)$_{25}$

Poly(A) binding buffer:
     0.5M LiCl, 10 mM Tris-Cl, pH 7.5, 1 mM EDTA, 0.1 % sodium dodecylsulphate.
Middle wash buffer:
     0.15M LiCl, 10 mM Tris-Cl, pH 7.5, 1 mM EDTA, 0.1 % sodium dodecylsulphate.
Elution buffer: 2 mM EDTA, 0.1 % SDS. Depending on the subsequent use of the purified mRNA SDS may be omitted in the last washing step and in the elution buffer.

Example 6

Removal of 3 bp from the ricin A gene by in vitro mutagenesis on magnetic particles

A cloned BamHI fragment containing the plant toxin gene coding for ricin A (Sundan et al., Nucleic Acid Res. 17, 4, 98-9) was ligated into the BamHI site in pBR322, creating the plasmid pALR 500 containing the sequence 2 pmol of this plasmid in 50 µl High salt buffer:
     100 mM NaCl
     50 mM Tris HCl (pH 7.5)
     10 mM MgCl$_2$
     1 mM DTT
was cut with the restriction enzymes EcoRI and SalI giving rise to two fragments.

The enzymes were inactivated by phenol extraction followed by EtOH precipitation (Maniatis).

6 units of Klenow enzyme and 2 nmol Biotin $^{21}$-dUTP (clontec) as the only nucleotide in RT for 1 hour.

This incorporates Biotin only at the SalI site. Free Biotin dUTP is removed with a sephadex G50 spin column treatment.

1 mg of streptavidin coated magnetic beads (Dynabeads 280) was added and placed on a roller for 15-30' RT, washed once in TE buffer (10 mM tris - 1 mM EDTA pH 7.5) and treated with 100 µl of 0.2 M NaOH for 2 minutes to separate the DNA strands and the beads are washed once with TE. Then the buffer was removed.

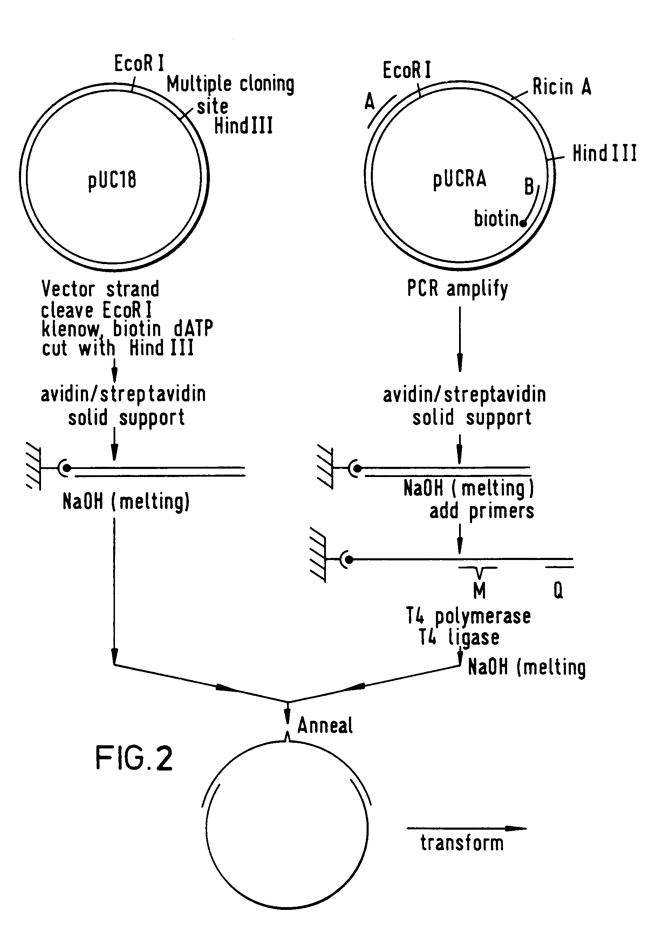

We ended up with single stranded DNA bound to the beads, Fig. 2:-

```
                  3'                  Ricin  A                        HindIII
        Support ———————————+—————————————————+——————————————+———————
                       BamHI                      BamHI            EcoRI
```

This is the single stranded template for the in vitro mutagenesis containing the non-coding strand of ricin A.

Primers used for the 1.strand synthesis:

1)

$$5' \ CAC \ GAT \ GCG \ TCC \ GGC \ GTA \ GA \ 3'$$

2) Mutation primer:

5'CC AAT GCA TAT GTG GTC GGC template contains CGT here TAC GCT GGA AAT AAT AGC CA TA 3'

Primer used for the second strand synthesis 3)

$$5'CAC \ TAT \ CGA \ CTA \ CGC \ GAT \ CA3'$$

Location of the primers is shown below

```
                  3'         BamHI                          BamHI     HindIII
        Support ———————————+————————————————————————+———————+——————
                               Ricin  A
                      5'——3'          5'——3'
                       (1)             (2)                       EcoRI
```

Start of in vitro mutagenesis

20 pmol of each primer 1) and phosphorylated mutation primer 2) in 20 µl 2 x SSC were added to the 1 mg of template beads/ Heated to 70°C and allowed to cool to room temperature.

To the hybridization mixture the following were added:

100 mM tris-Cl pH 8.8

20 µl 5 x polymerase mix 10 mM DTT

2.5 U T4 DNA polymerase 50 mM MgCl$_2$

2 U T4 DNA ligase 2.5 mM each of dATP,

H$_2$O to 100 µl dGTP, dTTP, dCTP

5 mM ATP

Mix

Incubate 5' at 0°C, then 5' at RT and then 2 hrs at 37°C.

The units of T4 polymerase may be more dependent of the quality of the enzyme.

The beads were washed once in H$_2$O and treated with 100 µl 0.2 M NaOH for 2 min. Then magnetic separation of the template coupled to the bead and the newly synthesized mutated 1. strand.

The supernatant was spun in a sephadex G50 spin column equilibrated with Klenow buffer.

20 pmol of primer 3) was added, heated to 70°C and allowed to cool to RT. 6 units of Klenow enzyme and 2 nmol of the fours dNTP's were added and incubated at RT for 60'.

The BamHI part of this newly synthesized ds. mutated fragment was cloned in puc 8 and positive clones were identified and sequenced.

Example 7

In vitro mutagenesis on magnetic particles

Materials and methods

Bacterial strains and plasmids.

E. coli RR1ΔM15 was used as bacterial host. the plasmid vector was pRIT28. Plasmid containing bacteria were grown on TBAB-plates containing ampicillin (200 μg/ml), X-gal and IPTG.

Enzymes and oligonucleotides.

Restriction endonucleases, T4 polynucleotide kinase, T4 DNA ligase and Klenow were purchased from Pharmacia, Sweden. T4 DNA polymerase was obtained from New England Biolabs and biotin-16-dUTP was obtained from Boehringer Mannheim, W. Germany. DNA manipulations and purifications were performed according to standard procedures. Oligonucleotide primers were synthesized by phosphoramidite chemistry on an automated DNA synthesis machine. (Gene Assembler, Pharmacia, Sweden).

Method

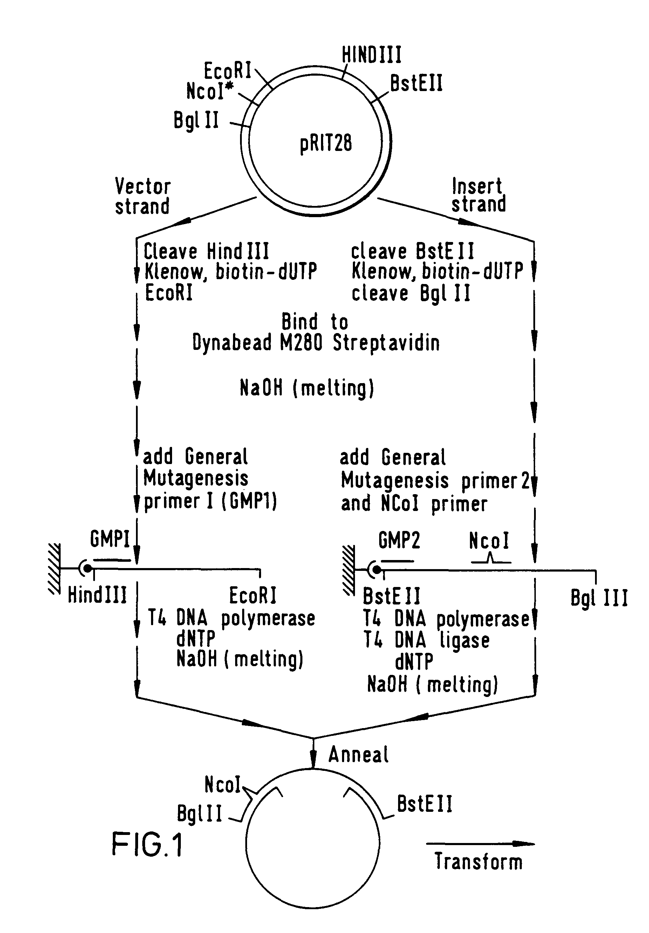

The protocol for solid phase in vitro mutagenesis outlined in Figure 1 was followed to introduce a unique NcoI-site in the lacZ' gene of plasmid vector pRIT28. This was performed using two general mutagenesis primers (GMPI and GMP2) complimentary to regions close to the HindIII-site and BstEII-site of pRIT28, and respective mutagenesis primers (NcoI-primer) complementary to a region upstream EcoRI except for one mismatch creating an NcoI restriction site at the second methionine codon in the lacZ' gene.

To yield the vector template, 10 μg of pRIT28 was digested with HindIII in a total volume of 50 μl. The 5' protruding ends were filled in using Klenow polymerase (5U). 1.5 μl biotin-16-dUTP (1 mM), 3 μl each of the appropriate dNTP's (0.5 mM), 7.5 μl of a buffer containing 100 mM Tris-HCl (pH7.5), 100 mM MgCl$_2$ and 1M NaCl. The volume of the reaction mixture was adjusted to 75 μl with water. The reaction was performed at 37°C during one hour. The material was purified using a Sephadex G50 column (Pharmacia, Sweden) followed by ethanol precipitation. After redissolving in TE (10 mM Tris pH 7.5, 1 mM EDTA) the plasmid was digested with EcoRI. This reaction mixture containing the biotinylated double stranded DNA was mixed with Dynabeads M280-Streptavidin previously washed with 1M NaCl and TE. The immobilized double stranded DNA was converted into single stranded form by melting off the coding strand by incubation at 37° with 0.15 M NaOH for 10 minutes.

To yield the "insert" template 10 μg of pRIT28 was digested with BstEII and the 5' protruding ends were biotinylated as above. After fill in, purification the plasmid was digested with Bgl II and thereafter immobilized on magnetic beads as the previous template. The Dynabeads with immobilized templates were subsequently washed with 0.15 M NaOH and water.

Extension reactions.

The oligonucleotide primers used to create the vector parts of the plasmid are GMP1 (5'GGCACTGGCCGTCGTTTTACAACGTCGTGA-3') and GMP2 (5'AGCACTCCATTGTCATGGTT-CAGGCTGCGC-3'). They are complementary to regions downstream of HindIII and upstream BstEII in pRIT28 respectively. The site specific mutagenesis was performed with a polynucleotide primer (5'-GTATTAAATTCG-TAACCATGGTCATAGCTG-3) that creates an NcoI restriction site at the second methionine codon in the lacZ' gene in pRIT28. This oligonucleotide is called NcoI-primer.

5 pmole of each oligonucleotide primer were annealed to their respective template immobilised on the Dynabead M280-streptavidin beads in a solution containing 10 mM Tris-HCl (pH7.5), 10 mM MgCl$_2$, 100 μg/ml BSA and 100 mM NaCl. The mixtures were incubated at 65° for a couple of minutes and allowed to cool to room temperature.

Extension reactions were performed by adding 1 μl BSA (1.00 g/ml), 6 μl polymerase mix (100 mM Tris HCl pH8.8, 10 mM DTT, 50 mM MgCl$_2$ and 5mM ATP), 6 μl chase (10mM of each dNTP) and 3.5U T4 DNA polymerase. 1U of T4 DNA ligase was added to the beads containing the insert strand. The volume was adjusted to 30 μl with water. The mixtures were incubated at room temperature for 20 minutes followed by incubation at 37°C during two hours. After extension the beads were washed once with TE. The newly synthesized strands were melted off by incubation with 20 μl 0.15 M NaOH at 37°C during 10 minutes. The pH of the supernatants were immediately adjusted with 1.5 µl HAc (1.7 M) and 2.2 µl 10xTE (100 mM Tris pH7.5 10mM EDTA).

The two supernatants were mixed and incubated at 70°C for 10 minutes and allowed to cool to room temperature. After annealing of the two strands, the $CaCl_2$ concentration was adjusted to 0.1M and E.coli RRIΔM15 was transformed with the DNA and spread on TBAB plates containing IPTG and X-gal.

As no direct selection method was available, screening of clones was done by restriction mapping of prepared plasmids, 20 clones were tested, 19 of these were positive, 3 of the positive clones were confirmed by DNA sequencing.

## Example 8

### In vitro mutagenesis on latex particles.

The protocol shown in Figure 2 was used

(a) To yield the ss vector template 10 µg of pUC18 were digested with EcoRI in a total volume of 50 µl. The 5'protruding ends were filled in using Klenow polymerase (5 U) and 2 µl Biotin-7-dATP (BRL), 7.5 µl of a buffer containing 100 mM Tris-HCl (pH 7.5), 100 mM $MgCl_2$ and 1M NaCl. The volume was adjusted to 75 µl with water. The reaction was performed at room temperature during one hour and after that purified using a sephadex G50 spin column. The purified biotinylated linearized vector was cut with HindIII. The reaction mixture containing the biotinylated doublestranded DNA was mixed with previously washed Pandex avidin particles, (Baxter Healthcare Corp., Mundelein, Illinois, USA)

To yield the ss vector template the immobilized doublestranded DNA was converted into singlestranded form by melting off the non attached strand by incubation at 37°C with 20 µl 0.15 M NaOH for 10 minutes. The pH of the supernatant was immediately adjusted with 1.5 µl HAc (1.7M) and 2.2 µl 10 x TE (100 mM tris pH 7.5, 10 mM EDTA).

(b) To yield the mutagenesis template, the inserted fragment from pUCRA was PCR amplified using 10 pmol of primer A TGC-TTC-CGG-CTC-GTA-TGT-TGT-GTG3' and biotinylated primer B Biotin-AAA-GGG-GGA-TGT-GCT-GCA-AGG-CGA3' in 25 µl PCR reaction mixture as recommended by Perkin Elmer and amplified for 20 cycles. After PCR amplification, Pandex avidin particles were added to immobilize the amplified insert with flanking vector sequences.

(c) To yield template for in vitro mutagenesis the immobilized PCR amplified fragment was made single stranded with 0.15M NaOH for 10 minutes at room temperature. 10 pmol were added of each primer Q 5'CGG-CTC-GTA-TGT-TGT-GTG-GAA-TTG and mutagenesis primer M 5'CC-AAT-GCA-TAT-GTG-GTC-GGC-TAC-GCT-GGA-AAT-AGC-GCA-TAT-TTC3' (the original sequence was: CCAAT GCA-TAT-GTG-GTC-GGC-TAC CGT GCT GGA AAT AGC- GCA) were annealed to the template immobilized on the Pandex avidin particles in a solution containing 10 mM Tris-HCl (pH 7.5), 10 mM $MgCl_2$, 100 µg/ml BSA and 100 mM NaCl. The mixture was incubated at 65°C for a few minutes and allowed to cool to room temperature.

(d) Extension was performed by adding 1 µl BSA (100 µg/ml), 6 µl polymerase mix (100 mM Tris-HCl pH 8.8, 10 mM DDT), 50 mM $MgCl_2$ and 5 mM ATP), 6 µl chase (10 mM each of dNTP) and 3.5 U $T_4$ DNA polymerase. 1 unit of $T_4$DNA ligase was added to the beads containing the insert strand.

The volume was adjusted to 30 µl with water. The mixture was incubated at RT for 20 minutes followed by incubation on a roller mixer at 37°C during two hours.

(e) After extension the beads were washed once with TE.

The newly synthesized strands were melted off by incubation with 20 µl 0.15M NaOH at 37°C during 10 minutes. The pH of the supernatant was immediately adjusted with 1.5 µl HAc (1.7M) and 2.2 µl 2 x TE.

The two supernatants, the single stranded vector and the newly mutated insert with flanking vector sequences were mixed and incubated at 70°C for 10 minutes and allowed to cool to RT.

After annealing of the two strands the $CaCl_2$ concentration was adjusted to 0.1M and E. coli DH5α was transformed with DNA and spread on TBAB plates containing IPTG and X-Gal.

## REFERENCES

1. Gilham, P.T. (1968) Biochemistry 7, 2809-2813.
2. Rickwood, D. (1972) Biochem. Biophys. Acta 269, 47-50.
3. Bünemann, H., Westhoff, P., and Herrmann, R.G. (1982) Nucl. Acids Res. 10, 7163 -7180.
4. Langdale, J.A., and Malcolm, A.D.B. (1984) Biochem.
    Soc. Trans. 12, 693 - 694.

5. Chu, B.C.F., Wahl, G.M., and Orgel, L.E. (1983) Nucl. Acids Res. 11, 6513 - 6529.

6. Ghosh, S.S., and Musso, G.F. (1987) Nucl. Acids Res. 15, 5353 - 5372.

7. Maniatis, T., Fritsch, E.F., and Sambrook, J. (1982) Molecular Cloning, A Laboratory manual, Cold Spring Harbor Laboratory.

8. Chu, B.C.F., and Orgel, L.E. (1985) DNA 4, 327-331.

9. Clerici, L., Campagnari, F., de Rooij, J.F.M., and van Boom, J.H. (1979) Nucl. Acids Res. 6, 247-258.

10. Noyes, B.E., and Stark, G.R. (1975) Cell 5, 301 - 310.

11. Chu, B.C.F., and Orgel, L.E. (1985) Proc. Natl. Acad. Sci. USA 82, 963 - 967.

## Claims

1. A method for _in vitro_ mutagenesis of a nucleic acid to produce single stranded DNA containing one or more base alterations, which includes the steps of:

   a) preparing an insoluble support carrying a nucleic acid to be mutagenised;

   b) adding a DNA oligonucleotide primer, which has the desired base alteration(s) but sufficient homology to hybridise with the appropriate site on the said first nucleic acid and allowing the primer to bind thereto;

   c) hybridising where necessary a further primer corresponding to the end of the nucleic acid at which synthesis is to start and adding a polymerase or reverse transcriptase, and nucleotides to form a DNA strand having the desired alteration(s) and including a ligase to ligate the said DNA strand to the primer; and

   d) separating the first nucleic acid from the synthesised DNA strand.

2. A method as claimed in claim 1 wherein said nucleic acid to be mutagenised is attached to said insoluble support by hybridisation to a nucleic acid probe attached to said support.

3. A method as claimed in claim 1 or claim 2 wherein said nucleic acid probe is covalently attached to said insoluble support.

4. A method as claimed in any preceding claim wherein said insoluble support consists of magnetic particles.

5. A method as claimed in claim 4 wherein the magnetic particles are monodisperse and/or superparamagnetic.

6. A method as claimed in claim 4 or claim 5 wherein said magnetic particles are magnetically aggregated between step (d) and step (e).

7. A method as claimed in any preceding claim wherein step (a) comprises amplification of said nucleic acid to be mutagenised by the polymerase chain reaction using a primer which is attached or is provided with means for subsequent attachment to the insoluble support.

8. A method as claimed in any preceding claim which includes washing steps and changes of buffer to optimise hybridisation and enzyme activity.

9. A kit for _in vitro_ mutagenesis of nucleic acids comprising:

   (a) an insoluble support adapted to capture a nucleic acid to be mutagenised;

   (b) a polymerase or reverse transcriptase; and one or more of:

   (c) a primer to initiate synthesis of cDNA;

   (d) deoxynucleotides; and

   (e) appropriate buffers.

10. A kit as claimed in claim 9 which additionally comprises:

    (i) two PCR primers one of which carries an affinity binding molecule capable of binding to said support; and

    (ii) an alkaline solution for strand separation.

EP 0 448 609 B1

**Patentansprüche**

1. Verfahren zur in vitro-Mutagenese einer Nucleinsäure zur Erzeugung einer einzelsträngigen DNA, die eine oder mehrere Basenänderung(en) enthält, umfassend die Stufen:

   a) Herstellen eines unlöslichen Trägers, der die zu mutagenisierende Nucleinsäure trägt,

   b) Zugabe eines Oligonucleotid-DNA-Primers, der die gewünschte(n) Basenänderung(en) besitzt, aber ausreichend homolog ist, um mit der geeigneten Stelle auf der ersten Nucleinsäure zu hybridisieren und die Bindung des Primers daran zu gestatten,

   c) gegebenenfalls Hybridisieren eines weiteren Primers, entsprechend dem Ende der Nucleinsäure, an dem die Synthese beginnen soll, und Zugabe einer Polymerase oder reversen Transkriptase und von Nucleotiden, um einen DNA-Strang mit der/den gewünschten Änderung(en) zu bilden, und Aufnahme einer Ligase, um den DNA-Strang an den Primer zu ligieren, und

   d) Abtrennen der ersten Nucleinsäure von dem synthetisierten DNA-Strang.

2. Verfahren nach Anspruch 1, **dadurch gekennzeichnet, daß** die zu mutagenisierende Nucleinsäure an den unlöslichen Träger durch Hybridisieren an eine an den Träger gebundene Nucleinsäuresonde gebunden wird.

3. Verfahren nach Anspruch 1 oder 2, **dadurch gekennzeichnet, daß** die Nucleinsäuresonde kovalent an den Träger gebunden wird.

4. Verfahren nach einem der vorausgehenden Ansprüche, **dadurch gekennzeichnet, daß** der unlösliche Träger aus magnetischen Teilchen besteht.

5. Verfahren nach Anspruch 4, **dadurch gekennzeichnet, daß** die magnetischen Teilchen monodispers und/oder superparamagnetisch sind.

6. Verfahren nach Anspruch 4 oder 5, **dadurch gekennzeichnet, daß** die magnetischen Teilchen zwischen Stufe (d) und Stufe (e) magnetisch aggregiert werden.

7. Verfahren nach einem der vorausgehenden Ansprüche, **dadurch gekennzeichnet, daß** die Stufe (a) die Amplifikation der zu mutagenisierenden Nucleinsäure durch die Polymerase-Kettenreaktion unter Verwendeung eines Primers, der an den unlöslichen Träger gebunden ist oder mit Einheiten zur anschließenden Bindung an den unlöslichen Träger versehen ist, umfaßt.

8. Verfahren nach einem der vorausgehenden Ansprüche, **dadurch gekennzeichnet, daß** es Waschstufen und Pufferwechsel umfaßt, um die Hybridisierung und die Enzymaktivität zu optimieren.

9. Kit zur in vitro-Mutagenese von Nucleinsäuren, umfassend:

   (a) einen unlöslichen Träger mit der Eignung zum Einfangen einer zu mutagenisierenden Nucleinsäure,

   (b) eine Polymerase oder reverse Transkriptase, und einen oder mehrere der Bestandteile:

   (c) Primer, um die Synthese der cDNA zu initiieren,

   (d) Desoxynucleotide, und

   (e) geeignete Puffer.

10. Kit nach Anspruch 9, **dadurch gekennzeichnet, daß** es weiterhin

    (i) zwei PCR-Primer, von denen einer ein Molekül zur Affinitätsbindung mit der Fähigkeit zur Bindung an den Träger trägt, und

    (ii) eine alkalische Lösung zur Strangtrennung umfaßt.

**Revendications**

1. Procédé de mutagenèse in vitro d'un acide nucléique afin de produire un ADN monocaténaire contenant une ou plusieurs bases modifiées, qui comprend les étapes consistant à:

   (a) préparer un support insoluble portant un acide nucléique à mutagéniser;

   (b) ajouter une amorce oligonucléotidique qui possède la ou les altérations de bases souhaitées mais qui présente une homologie suffisante pour s'hybrider avec le site approprié dudit premier acide nucléique et à laisser l'amorce s'hybrider avec celui-ci;

14

(c) si nécessaire, hybrider une autre amorce correspondant à l'extrémité de l'acide nucléique à laquelle doit démarrer la synthèse et ajouter une polymérase ou une transcriptase reverse ainsi que des nucléotides afin de former un brin d'ADN possédant la ou les altérations désirées et une ligase qui lie ledit brin d'ADN à l'amorce; et

(d) séparer le premier acide nucléique du brin d'ADN synthétisé.

2. Procédé selon la revendication 1 dans lequel ledit acide nucléique à mutagéniser est attaché audit support insoluble par hybridation à une sonde d'acide nucléique attachée audit support.

3. Procédé selon la revendication 1 ou 2 dans lequel ladite sonde d'acide nucléique est attachée de manière covalente audit support insoluble.

4. Procédé selon l'une quelconque des revendications précédentes dans lequel le support insoluble est constitué de particules magnétiques.

5. Procédé selon la revendication 4 ou 5 dans lequel les particules magnétiques sont monodisperses et/ou super-paramagnétiques.

6. Procédé selon la revendication 4 ou 5 dans lequel les particules magnétiques sont magnétiquement agrégées entre l'étape (d) et l'étape (e).

7. Procédé selon l'une quelconque des revendications précédentes dans lequel l'étape (a) comprend l'amplification dudit acide nucléique à mutagéniser par la réaction de polymérisation en chaîne en utilisant une amorce qui est attachée ou munie de moyens lui permettant de s'attacher ultérieurement au support insoluble.

8. Procédé selon l'une quelconque des revendications précédentes qui inclut des étapes de lavage et de changement de tampon visant à optimiser l'hybridation et l'activité enzymatique.

9. Kit pour la mutagenèse in vitro des acides nucléiques comprenant
   (a) un support insoluble adapté à la capture d'un acide nucléique à mutagéniser;
   (b) une polymérase ou une transcriptase reverse et une ou plusieurs;
   (c) amorce destinée à initier la synthèse d'ADNc;
   (d) désoxynucléotide et
   (e) tampon approprié

10. Nécessaire selon la revendication 9 qui comprend en outre:
    (i) deux amorces de PCR dont l'une porte une molécule de liaison par affinité capable de se lier audit support; et
    (ii) une solution alcaline pour la séparation des brins.

FIG.1

FIG.2